# EUROPEAN PATENT APPLICATION

(11) **EP 4 748 346 A1**
(43) Date of publication of application: **27.05.2026**
(21) Application number: 24843341.9
(22) Date of filing: 24.06.2024
(51) Int. Cl.: A61C 19/04, A61C 9/00, A61B 5/00, A61C 5/00, A61C 7/00, G06F 30/00, G06T 17/00, G06T 1/00, A61C 19/05

(54) **DATA PROCESSING METHOD, DEVICE, AND RECORDING MEDIUM**

(30) Priority: 19.07.2023 KR 20230093877; 17.04.2024 KR 20240051500
(71) Applicant: Medit Corp., Seoul 07207 (KR)
(72) Inventor: LEE, Jungkyen, Seoul 07207 (KR); LEE, Hotaik, Seoul 07207 (KR)
(74) Representative: MFG Patentanwälte Meyer-Wildhagen Meggle-Freund Gerhard PartG mbB
(86) International application number: PCT/KR2024/008697
(87) International publication number: WO 2025/018621

(57) **Abstract**

According to an embodiment, a method performed by an electronic device includes: generating first data on an inner surface of an intraoral appliance, based on three-dimensional scan data on a surface of a subject; generating second data on an outer surface of the intraoral appliance, based on the first data; and generating third data on an intermediate surface of the intraoral appliance located between the inner surface and the outer surface, based on the first data and the second data.

## Description

### TECHNICAL FIELD

The present disclosure relates to data processing technology. More specifically, the present disclosure relates to technology for generating data on an intraoral appliance, based on three-dimensional scan data obtained by scanning a patient's intraoral structure using a three-dimensional scanner (3D scanner).

### BACKGROUND ART

A patient's intraoral structure may be recognized based on images obtained by inserting a three-dimensional scanner into a patient's oral cavity and scanning the oral cavity with the three-dimensional scanner. For example, a three-dimensional scanner may be inserted into a patient's oral cavity, and the teeth region, gingiva region, and soft tissue region may be scanned, thereby obtaining two-dimensional scan data on the patient's intraoral structure. By applying three-dimensional modeling technology to the obtained two-dimensional scan data, three-dimensional scan data of the patient's intraoral structure may be generated.

Meanwhile, an appliance capable of being mounted in the patient's oral cavity may be designed to alleviate or prevent disorders caused by the patient's intraoral structure or oral habits. For example, a patient having an intraoral structure with poor occlusion may easily experience fatigue in the temporomandibular joint and may further develop temporomandibular joint disorders. Further, an intraoral structure in which the dentition in the teeth region is irregular or the gums are protruded may cause bimaxillary prognathism. As described above, an appliance may be designed to temporarily fix the patient's dentition in a specific form so that the patient does not experience discomfort caused by his or her intraoral structure. Alternatively, an appliance may be designed to orthodontically adjust the patient's dentition in the long term so that the dentition attains a specific form. As another example, patients with oral habits, such as bruxism, may experience tooth damage. In this way, an appliance may be designed to protect the patient's teeth and to prevent the patient from experiencing discomfort or disorders caused by the patient's oral habits. As described above, intraoral appliances capable of being mounted in the patient's oral cavity for various purposes, such as tooth fixation, orthodontics, and tooth protection, may include splints. Alternatively, the intraoral appliance may include one or more components that can be assembled to form a single splint.

Typically, intraoral appliances are manufactured using a single material, which may cause some patients to experience difficulty in fitting and removing them. For example, if an intraoral appliance is made of a material that is not smooth or flexible, patients with sensitive gums may not only experience difficulty fitting and removing the intraoral appliance, but also experience persistent discomfort while wearing it.

Therefore, there is a need to fabricate intraoral appliances using two or more materials in order to further improve wearing comfort of an intraoral appliance.

### DISCLOSURE

### TECHNICAL PROBLEM

The present disclosure is directed to providing a technology for generating data on an intraoral appliance to which two or more materials may be applied, based on scan data on an intraoral structure obtained using a three-dimensional scanner.

Further, the present disclosure is directed to providing a technology for generating an intermediate surface between an inner surface and an outer surface of an intraoral appliance, and for generating data on the intraoral appliance to which two or more materials may be applied, based on the intermediate surface.

Further, the present disclosure is directed to providing a technology for generating data on a portion of an intraoral appliance to which different materials are applied, based on the intermediate surface located between the inner surface and the outer surface of the intraoral appliance.

Further, the present disclosure is directed to providing a technology for providing a printing device capable of generating an intraoral appliance with data on an intraoral appliance to which two or more materials may be applied.

### TECHNICAL SOLUTION

According to an embodiment, a method performed by an electronic device includes: generating first data on an inner surface of an intraoral appliance, based on three-dimensional scan data on a surface of a subject; generating second data on an outer surface of the intraoral appliance, based on the first data; and generating third data on an intermediate surface of the intraoral appliance located between the inner surface and the outer surface, based on the first data and the second data.

In an embodiment, the method performed by the electronic device further includes: identifying a first material as a substance of a first portion of the intraoral appliance between the inner surface and the intermediate surface; and identifying a second material as a substance of a second portion of the intraoral appliance between the intermediate surface and the outer surface.

In an embodiment, the first portion of the intraoral appliance accommodates one or more teeth within an oral cavity, the second portion of the intraoral appliance forms an outline of the intraoral appliance, and at least one of the first portion or the second portion of the intraoral appliance becomes thinner toward a gingival region within the oral cavity.

In an embodiment, the third data further includes data on locking of the first portion and the second portion.

In an embodiment, the first material is more flexible than the second material.

In an embodiment, the generating the third data on the intermediate surface includes: identifying a first point on the inner surface and a normal vector to the inner surface at the first point; identifying a first offset point spaced apart from the first point by a first offset value in a direction of the normal vector; and identifying a second point on the intermediate surface corresponding to the first point, based on whether the first offset point is located between the inner surface and the outer surface and a minimum distance between the first offset point and one or more points on the outer surface is greater than a predetermined value.

In an embodiment, the identifying the second point on the intermediate surface corresponding to the first point, based on whether the first offset point is located between the inner surface and the outer surface and the minimum distance between the first offset point and the one or more points on the outer surface is greater than the predetermined value, further includes: identifying the second point as being identical to the first offset point in response to the first offset point being located between the inner surface and the outer surface and the minimum distance between the first offset point and the one or more points on the outer surface being greater than the predetermined value.

In an embodiment, the identifying the second point on the intermediate surface corresponding to the first point, based on whether the first offset point is located between the inner surface and the outer surface and the minimum distance between the first offset point and the one or more points on the outer surface is greater than the predetermined value, includes: identifying a second offset point spaced apart from the first offset point by a second offset value in a direction opposite to the normal vector in response to the first offset point being located between the inner surface and the outer surface and to the minimum distance between the first offset point and the one or more points of the outer surface being less than or equal to the predetermined value; and identifying a second point on the intermediate surface corresponding to the first point, based on whether the second offset point is located between the inner surface and the outer surface and a minimum distance between the second offset point and the one or more points on the outer surface is greater than the predetermined value.

In an embodiment, the identifying the second point on the intermediate surface corresponding to the first point, based on whether the first offset point is located between the inner surface and the outer surface and the minimum distance between the first offset point and the one or more points on the outer surface is greater than the predetermined value, includes: identifying a third offset point spaced apart from the first offset point by a third offset value in a direction opposite to the normal vector in response to the first offset point not being located between the inner surface and the outer surface; and identifying the second point on the intermediate surface corresponding to the first point, based on whether the third offset point is located between the inner surface and the outer surface and a minimum distance between the third offset point and the one or more points on the outer surface is greater than the predetermined value.

In an embodiment, the method performed by the electronic device further includes: identifying a plurality of point pairs, each point pair including one point on the inner surface and one point on the outer surface; identifying a point pair having a closest distance between points from among the plurality of point pairs; and determining the first offset value, based on the identified point pair.

In an embodiment, the method performed by the electronic device further includes receiving a user input for the first offset value.

In an embodiment, the generating the third data on the intermediate surface includes: identifying a plurality of point pairs, each point pair including one point on the inner surface and one point on the outer surface; identifying a plurality of intermediate points based on the plurality of point pairs, each intermediate point positioned between one point on the inner surface and one point on the outer surface, which constitute a point pair; and generating the third data such that the intermediate surface includes the plurality of intermediate points.

In an embodiment, the method performed by the electronic device further includes: identifying a specific point having a shortest distance from a specific point on a periphery of the intermediate surface from among one or more points constituting a periphery of the inner surface; identifying a specific point having a shortest distance from a specific point on the periphery of the intermediate surface from among one or more points constituting a periphery of the outer surface; and modifying the third data so that the intermediate surface includes a specific point on a straight line connecting the specific point on the periphery of the inner surface and the specific point on the periphery of the outer surface.

In an embodiment, the modifying the third data includes: identifying a point located on a straight line connecting the specific point on the periphery of the inner surface and the specific point on the periphery of the outer surface, and spaced apart from the specific point on the periphery of the inner surface by a fourth offset value; and modifying the third data so that the intermediate surface includes the identified point.

In an embodiment, the method performed by the electronic device further includes: receiving a user input for moving a specific point on the intermediate surface; identifying, based on the user input, a point located on a straight line connecting a specific point on a periphery of the inner surface and a specific point on a periphery of the outer surface, and spaced apart from the specific point on the periphery of the inner surface; and modifying the third data so that the intermediate surface includes the identified point.

An electronic device according to the present disclosure may include one or more processors, and one or more memories configured to store instructions executed by the one or more processors, wherein, when the instructions are executed by the one or more processors, the one or more processors may be configured to execute the method according to the present disclosure.

A non-transitory computer-readable recording medium according to the present disclosure may have instructions recorded thereon, which, when executed by one or more processors, cause the one or more processors to perform an operation, and the instructions may be configured to cause the one or more processors to execute the method according to the present disclosure.

### ADVANTAGEOUS EFFECTS

According to the present disclosure, it is possible to generate data on an intraoral appliance to which two or more materials may be applied, based on scan data on an intraoral structure obtained using a three-dimensional scanner.

Further, according to the present disclosure, it is possible to generate an intermediate surface between the inner surface and the outer surface of an intraoral appliance, and to generate data on the intraoral appliance to which two or more materials may be applied, based on the intermediate surface.

Further, the present disclosure is capable of generating data on a portion of an intraoral appliance to which different materials are applied, based on the intermediate surface located between the inner surface and the outer surface of the intraoral appliance.

Further, the present disclosure is capable of providing a printing device capable of generating an intraoral appliance with data on an intraoral appliance to which two or more materials may be applied.

### DESCRIPTION OF DRAWINGS

FIG. 1 is a diagram illustrating a system for obtaining scan data using a three-dimensional scanner according to an embodiment of the present disclosure.
FIG. 2A is a block diagram of an electronic device and a three-dimensional scanner according to an embodiment of the present disclosure.
FIG. 2B is a perspective view of a three-dimensional scanner according to an embodiment of the present disclosure.
FIG. 3A is a diagram illustrating maxillary scan data and mandibular scan data of an intraoral structure according to an embodiment of the present disclosure.
FIG. 3B is a diagram illustrating bimaxillary scan data of an intraoral structure according to an embodiment of the present disclosure.
FIG. 4 is a diagram illustrating three-dimensional scan data on a surface of a subject according to an embodiment of the present disclosure.
FIG. 5 is a diagram illustrating an inner surface of an intraoral appliance according to an embodiment of the present disclosure.
FIG. 6 is a diagram illustrating an outer surface of an intraoral appliance according to an embodiment of the present disclosure.
FIG. 7A is a diagram illustrating an intraoral appliance according to an embodiment of the present disclosure.
FIG. 7B is a cross-sectional view of the intraoral appliance in FIG. 7A.
FIG. 8 is a flowchart illustrating a data processing method according to an embodiment of the present disclosure.
FIG. 9A is a diagram illustrating a method for generating an intermediate surface of an intraoral appliance according to an embodiment of the present disclosure.
FIG. 9B is a diagram illustrating a method for generating an intermediate surface of an intraoral appliance according to an embodiment of the present disclosure.
FIG. 10A is a diagram illustrating a periphery of an intermediate surface of an intraoral appliance according to an embodiment of the present disclosure.
FIG. 10B is a diagram illustrating a periphery of an intermediate surface of an intraoral appliance according to an embodiment of the present disclosure.
FIG. 11A is a diagram illustrating a method for modifying an intermediate surface of an intraoral appliance according to an embodiment of the present disclosure.
FIG. 11B is a diagram illustrating a method for modifying an intermediate surface of an intraoral appliance according to an embodiment of the present disclosure.
FIG. 12A is a diagram illustrating an intraoral appliance according to an embodiment of the present disclosure.
FIG. 12B is a cross-sectional view of the intraoral appliance in FIG. 12A.
FIG. 12C is a cross-sectional view of the intraoral appliance in FIG. 12A.
FIG. 12D is a cross-sectional view of the intraoral appliance in FIG. 12A.
FIG. 13 is a diagram illustrating an intraoral appliance mounted to a subject according to an embodiment of the present disclosure.
FIG. 14 is a diagram illustrating a method for generating an intraoral appliance according to an embodiment of the present disclosure.

### MODE FOR INVENTION

The various embodiments described in this document are presented for the purpose of clearly explaining the technical idea of the present disclosure, and are not intended to limit the present disclosure to any particular embodiment. The technical idea of the present disclosure includes various modifications, equivalents, and alternatives of the respective embodiments described in the present document, as well as embodiments selectively combined from all or some of the respective embodiments. In addition, the scope of the technical idea of the present disclosure is not limited to the various embodiments presented below or the specific descriptions thereof.

Unless otherwise defined, the terms used in this document, including technical or scientific terms, may have meanings that are generally understood by those skilled in the art to which the present disclosure pertains.

Expressions such as "include," "may include," "comprise," "may comprise," "have," and "may have" used herein indicate the presence of the corresponding features (e.g., functions, operations, components, etc.) and do not preclude the presence of additional features. That is, such expressions are to be understood as open-ended terms that imply the possibility of including other embodiments.

A singular form used in this document may include plural forms unless the context clearly indicates otherwise, and this likewise applies to singular expressions recited in the claims.

Expressions such as "first," "second," or "primary," "secondary," and the like, used in this document, are employed merely to distinguish one element from another among a plurality of elements, unless the context clearly indicates otherwise, and do not limit the order or importance of the elements.

Expressions such as "A, B, and C," "A, B, or C," "A, B, and/or C," "at least one of A, B, and C," "at least one of A, B, or C," "at least one of A, B, and/or C," "at least one selected from A, B, and C," "at least one selected from A, B, or C," and "at least one selected from A, B, and/or C," as used in the present document, may refer to each of the listed items or any possible combination of the listed items. For example, the expression "at least one selected from A and B" may refer to (1) A, (2) at least one of A, (3) B, (4) at least one of B, (5) at least one of A and at least one of B, (6) at least one of A, and B, (7) at least one of B, and A, or (8) both A and B.

The expression "based on" or "according to," as used in the present document, is employed to describe one or more factors that influence a decision, determination, action, or operation described in the phrase or sentence in which the expression appears, and does not exclude the presence of additional factors influencing such decision, determination, action, or operation.

The expressions "one component (e.g., a first component) is 'connected' or 'coupled' to another component (e.g., a second component)," as used in the present document, may refer not only to the first component being directly connected or coupled to the second component, but also to the first component being connected or coupled to the second component indirectly through another component (e.g., a third component).

The expression "configured to," as used in the present document, may have meanings such as "set to," "capable of," "modified to," "designed to," or "able to," depending on the context. The expression is not limited to meaning "specifically hardware-designed," and for example, a processor configured to perform a specific operation may refer to a special-purpose computer structured through programming so as to perform the specific operation.

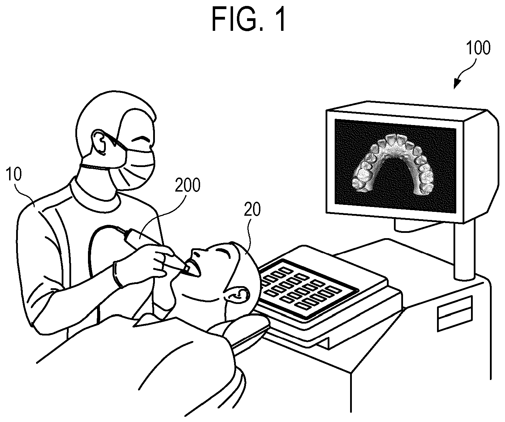

FIG. 1 is a diagram illustrating a system for obtaining scan data using a three-dimensional scanner 200 according to an embodiment of the present disclosure.

According to an embodiment, the three-dimensional scanner 200 may be a dental medical device for obtaining scan data on a surface of a subject 20. Here, the surface of the subject 20 may be an oral cavity surface of the subject 20.

For example, the three-dimensional scanner 200 may be an intraoral scanner.

For example, a user 10 (e.g., a dentist or dental hygienist) may obtain scan data on an intraoral structure of the subject 20 from the subject 20 (e.g., a patient) using the three-dimensional scanner 200.

For example, the user 10 may also obtain an image of the intraoral structure of the subject 20 from a diagnostic model (e.g., a plaster model or an impression model) modeling the shape of the intraoral structure of the subject 20.

For convenience of explanation, the following description describes obtaining scan data on the intraoral structure of the subject 20 by scanning the intraoral structure of the subject 20, but the present disclosure is not limited thereto, and it is also possible to obtain scan data on other regions of the subject 20.

For example, the three-dimensional scanner 200 may be a handheld scanner that may be inserted into and removed from the oral cavity and that has a scanning distance and angle, which may be freely adjusted by the user 10.

In an embodiment, the three-dimensional scanner 200 may be inserted into the oral cavity of the subject 20 and may scan the oral cavity in a non-contact manner, thereby obtaining scan data on the intraoral structure.

For example, the scan data on the intraoral structure may represent an image including a teeth region including at least one tooth, a gingival region, an artificial structure insertable into the oral cavity, and the like. Here, the artificial structure may include an orthodontic device including brackets and wires, an implant, a denture, an orthodontic auxiliary device inserted into the oral cavity, a splint, etc.

For example, the three-dimensional scanner 200 may irradiate light into the oral cavity of the subject 20 using a light source (or projector). As a specific example, the three-dimensional scanner 200 may irradiate light to at least a portion of the oral cavity, such as the teeth region or gingival region, and receive light reflected from the oral cavity of the subject 20 through a camera (or image sensor). As another example, the three-dimensional scanner 200 may obtain scan data on the intraoral structure by scanning an oral diagnostic model. If the oral diagnostic model is a diagnostic model modeled after the intraoral structure of the subject 20, the scan data on the oral diagnostic model may be scan data on the intraoral structure of the subject 20. For convenience of explanation, the following description assumes that scan data on the intraoral structure is obtained by scanning the oral cavity of the subject 20, but the present disclosure is not limited thereto.

In an embodiment, the three-dimensional scanner 200 may obtain two-dimensional scan data on the intraoral structure of the subject 20, based on information received through the camera. Here, the two-dimensional scan data on the intraoral structure may represent a two-dimensional image of the intraoral structure.

For example, the two-dimensional scan data on the intraoral structure of the subject 20 may represent a two-dimensional image including the teeth region, gingival region, artificial structure, tongue, etc. of the oral cavity of the subject 20.

In an embodiment, the two-dimensional scan data on the intraoral structure obtained from the three-dimensional scanner 200 may be transmitted to the electronic device 100 via a wired or wireless communication network.

For example, the electronic device 100 may be a computer device or a portable communication device. The electronic device 100 may generate three-dimensional scan data on the intraoral structure, which represents the intraoral structure three-dimensionally, based on the two-dimensional scan data on the intraoral structure received from the three-dimensional scanner 200. As a specific example, the electronic device 100 may three-dimensionally model the intraoral structure, based on the received two-dimensional scan data of the intraoral structure, thereby generating three-dimensional scan data of the intraoral structure.

In an embodiment, the three-dimensional scanner 200 may scan the intraoral structure of the subject 20 to obtain two-dimensional scan data on the intraoral structure and, based on this, generate three-dimensional scan data on the intraoral structure. That is, the three-dimensional scanner 200 may generate three-dimensional scan data on the intraoral structure and transmit it to the electronic device 100.

In an embodiment, the electronic device 100 may be connected to a cloud server (not shown) or a database (not shown).

For example, the electronic device 100 may transmit the two-dimensional scan data or three-dimensional scan data on the intraoral structure of the subject 20 to the cloud server or database, and the cloud server or database may store the two-dimensional scan data or three-dimensional scan data on the intraoral structure of the subject 20 received from the electronic device 100.

Although the three-dimensional scanner 200 has been described above as a handheld scanner, the present disclosure may also be applied to a table scanner that is fixed in a specific location. That is, the method proposed in the present disclosure may also be implemented using a table scanner. The table scanner may generate three-dimensional scan data on an oral diagnostic model by scanning the oral diagnostic model. Since the light source (or projector) and camera of the table scanner are fixed, the user 10 may scan the oral diagnostic model by moving the oral diagnostic model.

In an embodiment, the electronic device 100 may be communicatively connected to a printing device 1400.

For example, the electronic device 100 may generate data on an intraoral appliance mountable in the oral cavity, based on a three-dimensional image of the intraoral structure, and transmit the data to the printing device 1400. The printing device 1400 may generate (print) the intraoral appliance, based on the data of the intraoral appliance. Here, the intraoral appliance may be an appliance (device) that is designed for various purposes, such as tooth fixation, orthodontics, and tooth protection of the patient, and is mountable in the oral cavity of the patient. For example, the intraoral appliance may be referred to as a dental anchoring device for fixing a patient's teeth, an orthodontic device for correcting a patient's teeth, or a dental protection device for protecting a patient's teeth. For example, the intraoral appliance may include a splint. Meanwhile, the present disclosure is not limited thereto, and the term "intraoral appliance" encompasses various types of devices capable of being mounted in the oral cavity. Even if a device is called by a word having the same or similar meaning, the embodiments of the present disclosure may be applied to such a device.

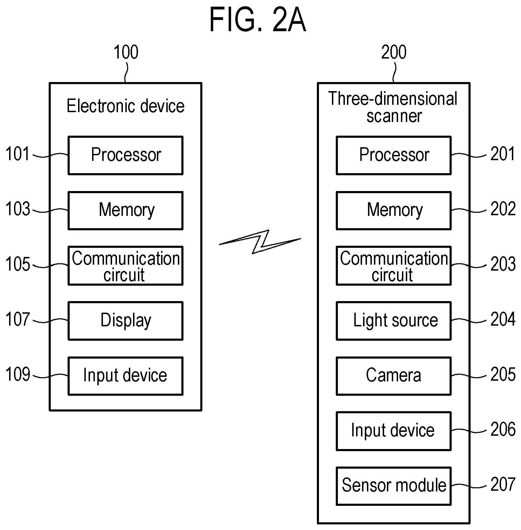

FIG. 2A is a block diagram of an electronic device 100 and a three-dimensional scanner 200 according to an embodiment of the present disclosure.

In an embodiment, the electronic device 100 and the three-dimensional scanner 200 may be connected to each other via a wired or wireless communication network and may transmit and receive various data to and from each other.

In an embodiment, the three-dimensional scanner 200 may include at least one of a processor 201, a memory 202, a communication circuit 203, a light source 204, a camera 205, an input device 206, or a sensor module (sensor) 207. At least one of the components included in the three-dimensional scanner 200 may be omitted, or other components may be added to the three-dimensional scanner 200. Additionally or alternatively, some components may be integrated or implemented as single or multiple entities. At least some components within the three-dimensional scanner 200 may be interconnected via a bus, a general-purpose input/output (GPIO), a serial peripheral interface (SPI), or a mobile industry processor interface (MIPI), thereby exchanging data and/or signals.

In an embodiment, the processor 201 of the three-dimensional scanner 200 is a component capable of performing operations or data processing related to the control or communication of the respective components of the three-dimensional scanner 200 and may be operatively connected to the components of the three-dimensional scanner 200. The processor 201 may load commands or data received from other components of the three-dimensional scanner 200 into the memory 202, process the commands or data stored in the memory 202, and store the resulting data.

In an embodiment, the memory 202 of the three-dimensional scanner 200 may store at least one instruction for the operation of the processor 201.

In an embodiment, the communication circuit 203 of the three-dimensional scanner 200 may establish a wired or wireless communication channel with an external device, including the electronic device 100, and transmit and receive various data to and from the external device.

For example, the communication circuit 203 may include at least one port for connecting to the external device via a wired cable to enable wired communication with the external device. In this case, the communication circuit 203 may communicate with the external device in a wired connection state through at least one port.

For example, the communication circuit 203 may be configured to include a cellular communication module so as to connect to a cellular network (e.g., 3G, LTE, 5G, WiBro, or WiMAX).

For example, the communication circuit 203 may include a short-range communication module to transmit and receive data to and from an external device, including the electronic device 100, using short-range communication (e.g., Wi-Fi, Bluetooth Low Energy (BLE), or UWB).

For example, the communication circuit 203 may include a contactless communication module for contactless communication. Specifically, the contactless communication may include at least one contactless proximity communication technology, such as near-field communication (NFC), radio frequency identification (RFID) communication, or magnetic secure transmission (MST) communication.

In an embodiment, the light source 204 of the three-dimensional scanner 200 may irradiate light toward the oral cavity of the subject 20.

For example, the light emitted from the light source 204 may be structured light having a predetermined pattern. As a specific example, the predetermined pattern may be a stripe pattern in which linear patterns of different colors appear continuously. The structured light pattern may be generated using a pattern mask or a digital micro-mirror device (DMD), but is not limited thereto.

In an embodiment, the camera 205 of the three-dimensional scanner 200 may obtain two-dimensional scan data regarding the intraoral structure of the subject 20 by receiving light reflected by the intraoral structure of the subject 20.

For example, the camera 205 may include a left camera corresponding to the left-eye field of view and a right camera corresponding to the right-eye field of view to construct three-dimensional scan data according to an optical triangulation method.

For example, the camera 205 may include at least one image sensor, such as a CCD sensor or a CMOS sensor.

In an embodiment, the input device 206 of the three-dimensional scanner 200 may receive user input for controlling the three-dimensional scanner 200.

For example, the input device 206 may include at least one of a button for receiving a push operation from the user 10, a touch panel for detecting a touch of the user 10, or a voice recognition device including a microphone.

For example, the user 10 may control starting or stopping of scanning using the input device 206.

In an embodiment, the sensor module 207 of the three-dimensional scanner 200 may detect an operating state of the three-dimensional scanner 200 or an external environmental state (e.g., a user's operation) and generate an electrical signal corresponding to the detected state.

For example, the sensor module 207 may include at least one of a gyro sensor, an acceleration sensor, a gesture sensor, a proximity sensor, or an infrared sensor.

For example, the user 10 may control the start or stop of scanning using the sensor module 207. As a specific example, in a case where the user 10 holds and moves the three-dimensional scanner 200, the processor 201 may control the three-dimensional scanner 200 to start a scanning operation when the angular velocity measured by the sensor module 207 exceeds a predetermined threshold value.

In an embodiment, the three-dimensional scanner 200 may start scanning in response to receiving a user input to start scanning through the input device 206 of the three-dimensional scanner 200 or the input device 109 of the electronic device 100.

In an embodiment, the three-dimensional scanner 200 may start scanning according to processing in the processor 201 of the three-dimensional scanner 200 or the processor 101 of the electronic device 100.

In an embodiment, when the user 10 scans the intraoral structure of the subject 20 through the three-dimensional scanner 200, the three-dimensional scanner 200 may generate two-dimensional scan data on the intraoral structure of the subject 20 and transmit the two-dimensional scan data on the intraoral structure of the subject 20 to an electronic device 100 in real time.

For example, the electronic device 100 may display a two-dimensional image of the intraoral structure of the subject 20 represented by the received two-dimensional scan data on the intraoral structure through the display 107.

For example, the electronic device 100 may generate three-dimensional scan data on the intraoral structure of the subject 20, based on the two-dimensional scan data on the intraoral structure of the subject 20. Further, the electronic device 100 may display, through the display 107, a three-dimensional image of the intraoral structure represented by the three-dimensional scan data on the intraoral structure of the subject 20. The electronic device 100 may also display the process of generating the three-dimensional scan data on the intraoral structure in real time through the display 107.

For example, the electronic device 100 may generate data on the intraoral appliance capable of being mounted in at least a portion of the intraoral structure of a subject 20, based on the three-dimensional scan data on the intraoral structure. Here, the data on the intraoral appliance may represent an image of the shape of the intraoral appliance. Further, the electronic device 100 may display the image of the shape of the intraoral appliance on the display 107. The electronic device 100 may also display the process of generating the data on the intraoral appliance in real time through the display 107.

In an embodiment, the electronic device 100 may include at least one of one or more processors 101, one or more memories 103, a communication circuit 105, a display 107, or an input device 109. At least one of the components included in the electronic device 100 may be omitted, or another component may be added to the electronic device 100. Additionally or alternatively, some components may be integrated or implemented as single or multiple entities. At least some components within the electronic device 100 may be interconnected via a bus, GPIO, SPI, MIPI, etc., to exchange signals or data.

In an embodiment, one or more processors 101 of the electronic device 100 may be configured to perform operations or data processing related to control or communication of the respective components of the electronic device 100.

For example, one or more processors 101 may be operatively connected to the components of the electronic device 100. One or more processors 101 may load commands or data received from other components of the electronic device 100 into one or more memories 103, process the commands or data stored in one or more memories 103, and store the resulting data.

In an embodiment, one or more memories 103 of the electronic device 100 may store at least one instruction for the operation of one or more processors 101.

For example, one or more memories 103 may store data (e.g., two-dimensional scan data or three-dimensional scan data on an intraoral structure) received from the three-dimensional scanner 200.

In an embodiment, the communication circuit 105 of the electronic device 100 may establish a wired or wireless communication channel with an external device, including at least one three-dimensional scanner 200, cloud server (not shown), or printing device 1400, and may transmit and receive various data to and from the external device.

For example, the communication circuit 105 may include at least one port for connecting to the external device via a wired cable in order to perform wired communication with the external device. In this case, the communication circuit 105 may communicate with the wired external device in a wired connection state through at least one port.

For example, the communication circuit 105 may be configured to include a cellular communication module so as to connect to a cellular network (e.g., 3G, LTE, 5G, WiBro, or WiMAX).

For example, the communication circuit 105 may include a short-range communication module to transmit and receive data to and from an external device, including at least one three-dimensional scanner 200, cloud server (not shown), or printing device 1400, using short-range communication (e.g., Wi-Fi, BLE, or UWB).

For example, the communication circuit 105 may include a contactless communication module for contactless communication. Specifically, the contactless communication may include at least one contactless proximity communication technology, such as NFC communication, RFID communication, or MST communication.

In an embodiment, the display 107 of the electronic device 100 may display various screens based on the control of the processor 101.

For example, the processor 101 of the electronic device 100 may control the components of the electronic device 100 to display two-dimensional scan data or three-dimensional scan data regarding the intraoral structure of the subject 20 through the display 107.

For example, the processor 101 may control the components of the electronic device 100 to display data regarding an intraoral appliance capable of being mounted in at least a portion of the oral cavity of the subject 20 through the display 107.

For example, an execution screen of a specific application may be displayed on the display 107 of the electronic device 100, and two-dimensional scan data or three-dimensional scan data on the intraoral structure may be displayed on the execution screen. Further, data on the intraoral appliance may be displayed on the execution screen. Here, a web browser or application for executing a specific application may be installed on the electronic device 100. The user 10 may edit, save, and delete the two-dimensional scan data or three-dimensional scan data on the intraoral structure displayed on the display 107 using the input device 109. Furthermore, the user 10 may edit, save, and delete the data on the intraoral appliance displayed on the display 107 using the input device 109.

In an embodiment, the input device 109 of the electronic device 100 may receive commands or data to be used in the components (e.g., one or more processors 101) of the electronic device 100 from an external source (e.g., the user 10).

For example, the input device 109 may be implemented in the form of a touch sensor panel that is connected to the display 107 and is capable of recognizing contact or proximity of various external objects.

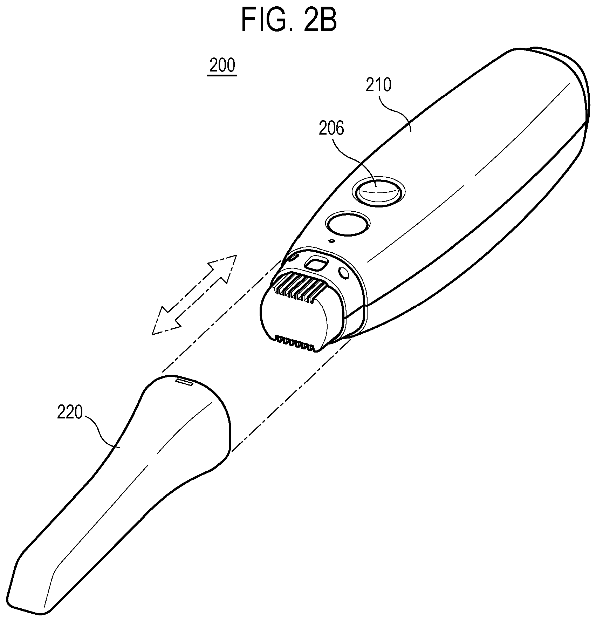

FIG. 2B is a perspective view of a three-dimensional scanner 200 according to an embodiment of the present disclosure.

In an embodiment, the three-dimensional scanner 200 may include a main body 210 and a probe tip 220.

For example, the main body 210 may have a shape configured to be easily held with the hand of the user 10.

For example, the probe tip 220 may have a shape configured to be easily inserted into and removed from the oral cavity of the subject 20.

For example, the main body 210 may be coupled to and separated from the probe tip 220.

For example, the components of the three-dimensional scanner 200 described in FIG. 2A may be provided within the main body 210.

For example, an opening may be formed at one end of the main body 210 to allow light output from the light source 204 to be irradiated into the oral cavity of the subject 20. Light irradiated through the opening may be reflected by the intraoral structure of the subject 20 and then re-enter through the opening. The reflected light entering through the opening may be captured by the camera 205 to generate two-dimensional scan data regarding the intraoral structure of the subject 20.

For example, the user 10 may initiate a scan using the input device 206 (e.g., a button) of the three-dimensional scanner 200. As a specific example, when the user 10 touches or presses the input device 206, light from the light source 204 may be irradiated onto the subject 20.

In an embodiment, the user 10 may scan the intraoral structure of the subject 20 while moving the three-dimensional scanner 200, and the three-dimensional scanner 200 may obtain two-dimensional scan data on the intraoral structure of the subject 20.

In an embodiment, the user may scan a diagnostic model for the oral cavity of the subject 20 while moving the three-dimensional scanner 200, and obtain two-dimensional scan data on the diagnostic model in this process. Here, the two-dimensional scan data on the diagnostic model may represent an image of the shape of the intraoral structure.

For example, the two-dimensional scan data on the intraoral structure may represent a two-dimensional image of a region including front teeth of the subject 20, a two-dimensional image of a region including molars of the subject 20, and the like.

For example, the three-dimensional scanner 200 may transmit the obtained two-dimensional scan data to the electronic device 100. In this case, the electronic device 100 may generate three-dimensional scan data on the intraoral structure, based on the received two-dimensional scan data on the intraoral structure. Here, the three-dimensional scan data on the intraoral structure may include at least one of maxillary scan data, mandibular scan data, or bimaxillary (occlusion) scan data regarding the intraoral structure.

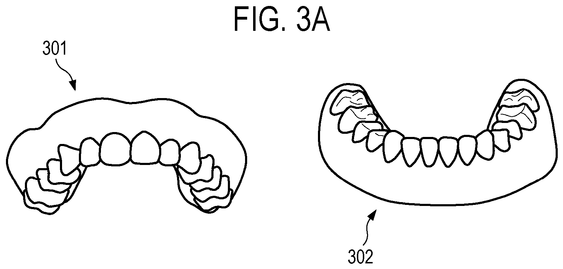

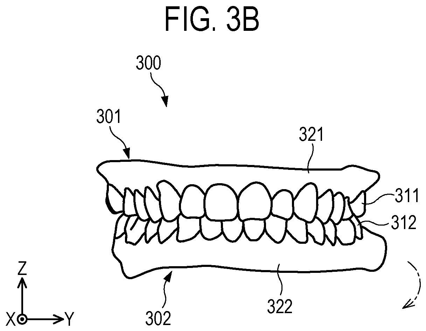

FIG. 3A is a diagram illustrating maxillary scan data 301 and mandibular scan data 302 of an intraoral structure according to an embodiment of the present disclosure. FIG. 3B is a diagram illustrating bimaxillary scan data 300 of an intraoral structure according to an embodiment of the present disclosure.

In an embodiment, the three-dimensional scan data on the surface of the subject 20 may include at least one of maxillary scan data 301, mandibular scan data 302, or bimaxillary scan data 300. Here, the surface of the subject 20 may include a surface of the intraoral structure of the subject 20.

In an embodiment, the maxillary scan data 301 may be three-dimensional scan data on the maxillary surface, which is generated based on two-dimensional scan data obtained as a result of scanning the maxillary surface within the oral cavity.

For example, the maxillary scan data 301 may represent a teeth region 311 and a gingival region 321 within the maxilla.

For example, based on the maxillary scan data 301, data on an intraoral appliance capable of being mounted on the maxillary surface of the oral cavity may be generated.

In an embodiment, the mandibular scan data 302 may be three-dimensional scan data on the mandibular surface, which is generated based on the two-dimensional scan data obtained as a result of scanning the mandibular surface within the oral cavity.

For example, the mandibular scan data 302 may represent a teeth region 312 and a gingival region 322 within the mandible.

For example, based on the mandibular scan data 302, data on an intraoral appliance capable of being mounted on the mandibular surface of the oral cavity may be generated.

In an embodiment, the bimaxillary scan data 300 may be three-dimensional scan data on the bimaxillary surface, which is generated based on the two-dimensional scan data obtained as a result of scanning the maxillary and mandibular surfaces within the oral cavity. Alternatively, the bimaxillary scan data 300 may be generated based on the maxillary scan data 301 and the mandibular scan data 302. That is, the bimaxillary scan data 300 may be scan data representing an image generated by synthesizing the image represented by the maxillary scan data 301 and the image represented by the mandibular scan data 302.

For example, based on the bimaxillary scan data 300, data on an intraoral appliance capable of being mounted on both the maxilla and the mandible of the oral cavity may be generated.

Hereinafter, a description of a data processing method proposed in the present disclosure will continue assuming that data on the intraoral appliance is generated based on the mandibular scan data 302. Meanwhile, this is presented solely for convenience of explanation, and the embodiments of the present disclosure are not limited thereto. That is, the description below may be equally applied even to the case where data on the intraoral appliance is generated based on the maxillary scan data 301 or the bimaxillary scan data 300.

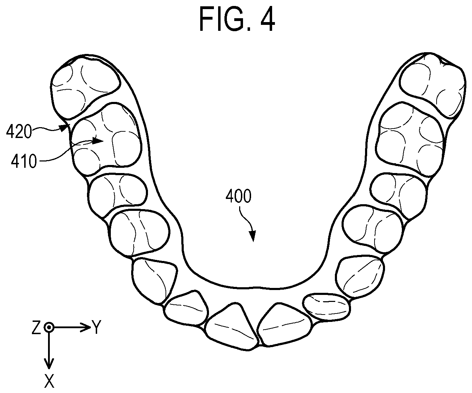

FIG. 4 is a diagram illustrating three-dimensional scan data 400 for a surface of a subject 20 according to an embodiment of the present disclosure.

The three-dimensional scan data 400 for the surface of the subject 20 in FIG. 4 may be mandibular scan data 302 for the surface of the mandible in the intraoral structure of the subject 20.

In an embodiment, the three-dimensional scan data 400 may represent at least a portion of the teeth region 410 or gingival region 420 in the mandible.

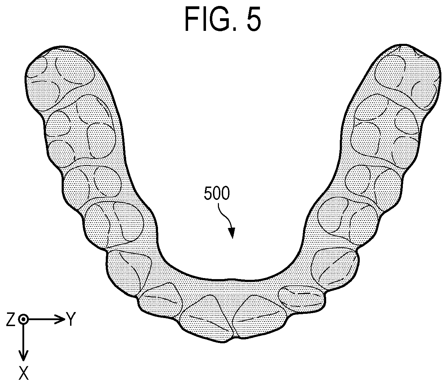

FIG. 5 is a diagram illustrating an inner surface 500 of an intraoral appliance according to an embodiment of the present disclosure.

In an embodiment, the electronic device 100 may generate the inner surface 500 of the intraoral appliance, based on three-dimensional scan data. That is, the electronic device 100 may generate first data on the inner surface 500 of the intraoral appliance, based on the three-dimensional scan data.

For example, the electronic device 100 may configure an occlusal plane and a midline for the three-dimensional scan data 400. Accordingly, the three-dimensional scan data 400 may be automatically aligned along the occlusal plane and aligned left and right by the midline.

For example, the user 10 may manually designate a reference point on the three-dimensional scan data 400 to configure the frontal direction of the three-dimensional scan data 400 and the occlusal plane, and align the three-dimensional scan data 400 along the configured occlusal plane. As a specific example, the user 10 may select some of the three-dimensional scan data 400 via the input device 109 of the electronic device 100 and align the three-dimensional scan data 400 based on the selected data as a reference point.

For example, the electronic device 100 may generate an inner surface 500 of an intraoral appliance based on the aligned three-dimensional scan data 400.

For example, the electronic device 100 may designate a direction in which the intraoral appliance is to be inserted toward at least a portion of the intraoral structure indicated by the three-dimensional scan data 400, based on an undercut in the aligned three-dimensional scan data 400. As a specific example, the electronic device 100 may designate a direction in which the intraoral appliance is to be inserted based on the area of the teeth region within the three-dimensional scan data 400, and an undercut and block-out according to the direction in which the intraoral appliance is to be inserted.

For example, the electronic device 100 may generate the inner surface 500 of the intraoral appliance based on an inner-surface offset value, surface smoothness, etc. As a specific example, the electronic device 100 may receive the inner-surface offset value, surface smoothness, etc. through the input device 109 and generate the inner surface 500 of the intraoral appliance based on the same. Here, the inner-surface offset value may be a separation distance between the three-dimensional scan data 400 and the intraoral appliance. Further, the surface smoothness may refer to roughness of the intraoral appliance.

For example, among the surfaces constituting the shape of the intraoral appliance, the inner surface 500 of the intraoral appliance may be a surface that contacts at least a portion of the teeth region 410 or gingival region 420 located in the direction in which the intraoral appliance is inserted toward at least a portion of the intraoral structure indicated by the three-dimensional scan data 400. That is, at least a portion of the teeth region 410 or gingival region 420 may be surrounded by the inner surface 500 of the intraoral appliance.

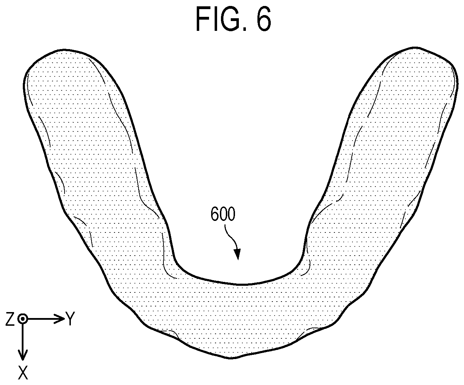

FIG. 6 is a drawing illustrating an outer surface 600 of the intraoral appliance according to an embodiment of the present disclosure.

In an embodiment, the electronic device 100 may designate the outline of the intraoral appliance based on the buccal height, the lingual height, etc. Here, the outline of the intraoral appliance may be a boundary line indicating the shape of the intraoral appliance.

For example, the electronic device 100 may receive the buccal height, the lingual height, etc. through the input device 109 and designate the outline of the intraoral appliance based on this. Here, the buccal height may be a height of the outer tooth wall facing the cheek based on the lower surface of the teeth region 410. As the buccal height increases, a formed buccal contour may be positioned closer to the gingival region 420. Additionally, the lingual height may be the height of the inner tooth wall facing the tongue based on the lower surface of the teeth region 410. As the lingual height increases, a formed lingual contour may be positioned closer to the gingival region 420.

In an embodiment, the electronic device 100 may generate an outer surface 600 of the intraoral appliance, based on the outline of the intraoral appliance, with respect to the aligned three-dimensional scan data 400. Here, among the surfaces constituting the shape of the intraoral appliance, the outer surface 600 of the intraoral appliance may be a surface that does not contact the teeth region 410 or the gingival region 420 located in the direction in which the intraoral appliance is inserted toward at least a portion of the intraoral structure represented by the three-dimensional scan data 400. That is, the outer surface 600 of the intraoral appliance is spaced from the teeth region 410 or the gingival region 420 when the intraoral appliance is actually mounted.

For example, the electronic device 100 may perform smoothing and flattening operations on an image generated based on the inner surface 500 and outline of the intraoral appliance, thereby generating an outer surface 600. Here, the smoothing operation may include Laplacian smoothing, which minimizes the surface area by minimizing the curvatures of curved surfaces on the outer surface 600 of the intraoral appliance. Further, the flattening operation may include generating a block hull for vertices within the image on which the smoothing operation has been performed using a convex hull algorithm, and correcting the vertices within the image according to the characteristics of the convex hull based on the generated block hull. The characteristics of the convex hull may be to move the position of a vertex by weighting its distance to the outermost surface of the block hull generated by the convex hull algorithm. That is, the image may be corrected based on a Bezier curve generated by varying the weights depending on the region for the vertices within the image where the smoothing operation was performed.

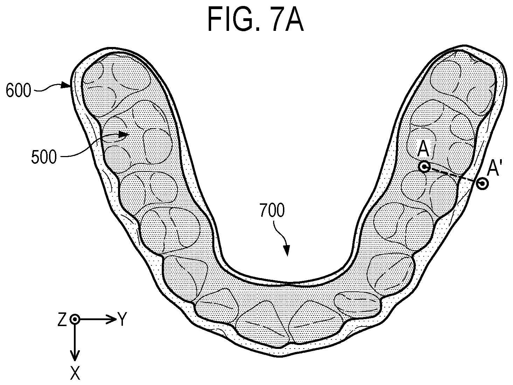

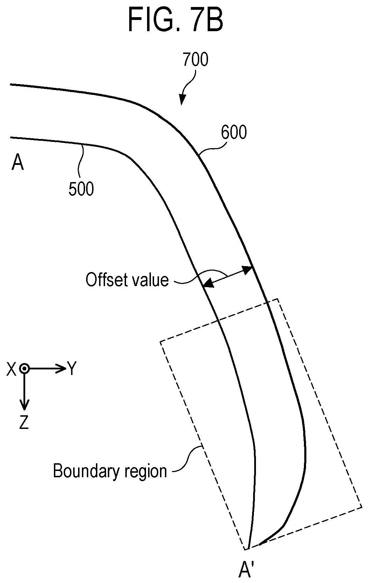

FIG. 7A is a diagram illustrating an intraoral appliance 700 according to an embodiment of the present disclosure. FIG. 7B is a cross-sectional view of the intraoral appliance 700 taken along line A-A' in FIG. 7A.

In an embodiment, the surface of the intraoral appliance 700 may include an inner surface 500 and an outer surface 600.

In an embodiment, the inner surface 500 and the outer surface 600 of the intraoral appliance 700 may be spaced apart from each other by an outer-surface offset value configured in the normal direction of the inner surface 500. Meanwhile, a boundary region (dotted line) may be configured with respect to the inner surface 500 and the outer surface 600 of the intraoral appliance 700. The distance between the vertex of the inner surface 500 and the vertex of the outer surface 600 within the boundary region may be configured to be smaller than the outer-surface offset value as it approaches the outline. Therefore, the distance between the inner surface 500 and the outer surface 600 may be reduced as the intraoral appliance 700 approaches the gingival region 420.

In an embodiment, the inner surface 500 of the intraoral appliance 700 may accommodate at least a portion of the teeth region 410 or gingival region 420 located in the direction in which the intraoral appliance 700 is inserted.

In an embodiment, the outer surface 600 of the intraoral appliance 700 may be formed to surround the inner surface 500 along the outline of the intraoral appliance 700.

Meanwhile, as illustrated in the region adjacent to A' in FIG. 7B, the inner surface 500 and the outer surface 600 of the intraoral appliance 700 may meet at an angle. Since the inner surface 500 and the outer surface 600 meet at an angle, the contact region between the periphery of the intraoral appliance 700 and the gingival region 420 in the region adjacent to A' may be formed at an angle and may be narrow, which may cause the wearer to experience discomfort or pain due to pressure being concentrated in the contact region. This concentration of pressure may cause persistent irritation or pressure on the gingiva, potentially leading to adverse effects such as damage or inflammation of surrounding tissues in the long term. In particular, since the contact region between the outer surface 600 and a specific region in the oral cavity (e.g., the frenulum region between the lip region and the gingival region) is large, if different materials are applied to the inner surface 500 and the outer surface 600, such as applying a material that is relatively harder than the inner surface 500 to the outer surface 600, while the contact region between the periphery of the intraoral appliance 700 and the gingival region 420 is formed narrowly, the wearer of the intraoral appliance 700 may feel a more distinct sense of foreignness in the contact region between the periphery of the intraoral appliance 700 and the gingival region 420. Thus, to improve the wearing comfort of the intraoral appliance 700, it is important to ensure that the inner surface 500 and outer surface 600 are smoothly connected to form a sufficiently wide and flat contact region between the periphery of the intraoral appliance 700 and the gingival region 420. This design optimizes the pressure distribution in the contact region between the periphery of the intraoral appliance 700 and the gingival region 420, thereby reducing wearer discomfort and minimizing irritation to oral tissues. Further, even when two or more materials are applied to the intraoral appliance 700, this design may help prevent the wearer from experiencing a sense of foreignness. Therefore, the present disclosure proposes a method for more conveniently producing an intraoral appliance 700 with excellent wearing comfort while applying two or more materials.

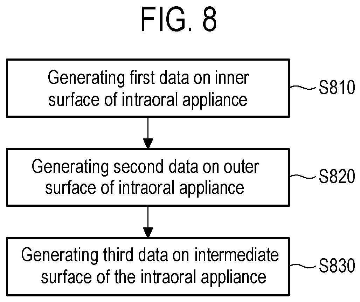

FIG. 8 is a flowchart of a data processing method according to an embodiment of the present disclosure.

In operation 810, the electronic device 100 may generate first data on the inner surface 500 of the intraoral appliance, based on three-dimensional scan data on the surface of the subject 20.

In an embodiment, the three-dimensional scan data on the surface of the subject 20 may include at least one of maxillary scan data 301 for the maxillary surface, mandibular scan data 302 for the mandibular surface, or bimaxillary scan data 300 for the bimaxillary surface in the oral cavity of the subject.

In an embodiment, the first data on the inner surface 500 of the intraoral appliance may include one or more points that constitute a shape of the inner surface 500 of the intraoral appliance. That is, the inner surface 500 of the intraoral appliance may include one or more points. Therefore, the operation of generating first data on the inner surface 500 of the intraoral appliance may be expressed as an operation of generating the inner surface 500 of the intraoral appliance.

In an embodiment, the electronic device 100 may configure an occlusal plane and a midline for three-dimensional scan data on the surface of a subject 20, and align the three-dimensional scan data according to the occlusal plane and midline.

In an embodiment, the electronic device 100 may determine a direction in which the intraoral appliance is to be inserted, based on an undercut of the three-dimensional scan data on the surface of the subject 20.

In an embodiment, the electronic device 100 may determine an inner-surface offset value, which indicates a separation distance between the three-dimensional scan data on the surface of the subject 20 and the inner surface 500 of the intraoral appliance. That is, each point indicated by the three-dimensional scan data on the surface of the subject 20 and each point on the inner surface 500 of the intraoral appliance may be spaced apart by the inner-surface offset value.

For example, the electronic device 100 may receive an inner-surface offset value from the user 10 via the input device 109.

In an embodiment, the electronic device 100 may generate first data regarding the inner surface 500 of the intraoral appliance, based on at least one of the aligned three-dimensional scan data, the direction in which the intraoral appliance is to be inserted toward at least a portion of the intraoral structure, or the inner-surface offset value.

In operation 820, the electronic device 100 may generate second data regarding the outer surface 600 of the intraoral appliance, based on the first data on the inner surface 500 of the intraoral appliance.

In an embodiment, the second data on the outer surface of the intraoral appliance may include one or more points that constitute a shape of the outer surface of the intraoral appliance. That is, the outer surface of the intraoral appliance may include one or more points. Therefore, the operation of generating the second data on the outer surface 600 of the intraoral appliance may be expressed as the operation of generating the outer surface 600 of the intraoral appliance.

In an embodiment, the electronic device 100 may determine an outline of the intraoral appliance, based on the shape, a buccal height, or a lingual height of the inner surface 500 of the intraoral appliance.

For example, the electronic device 100 may determine the buccal height of the intraoral appliance. As a specific example, the electronic device 100 may receive input of the buccal height from the user 10 via the input device 109.

For example, the electronic device 100 may determine the lingual height of the intraoral appliance. As a specific example, the electronic device 100 may receive input of the lingual height from the user 10 via the input device 109.

In an embodiment, the electronic device 100 may generate second data on the outer surface 600 of the intraoral appliance, based on the outline of the intraoral appliance.

For example, the electronic device 100 may perform a smoothing or flattening operation on the outer surface 600 of the intraoral appliance.

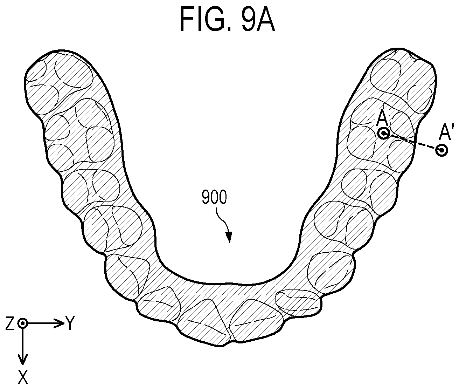

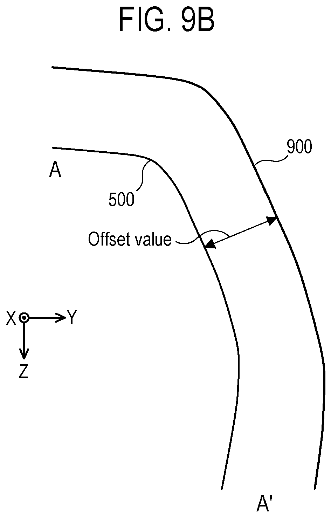

In operation 830, the electronic device 100 may generate third data on an intermediate surface 900 of the intraoral appliance, based on first data on the inner surface 500 of the intraoral appliance and the second data on the outer surface 600. Here, the intermediate surface 900 of the intraoral appliance may be an entity generated to be positioned between the inner surface and the outer surface of the intraoral appliance. For example, the intermediate surface of the intraoral appliance may be a virtual surface generated to be positioned between the inner surface and the outer surface of the intraoral appliance. Hereinafter, a method for generating the intermediate surface of the intraoral appliance will be described in detail with reference to FIGS. 9A and 9B.

FIGS. 9A and 9B are diagrams illustrating a method for generating an intermediate surface 900 of the intraoral appliance according to an embodiment of the present disclosure. FIG. 9B is a cross-sectional view of an inner surface 500 and an intermediate surface 900 of the intraoral appliance taken along line A-A', according to an embodiment of the present disclosure.

In an embodiment, the electronic device 100 may generate an intermediate surface 900 of the intraoral appliance, positioned between the inner surface 500 and the outer surface 600 of the intraoral appliance. Here, the shape of the intermediate surface 900 of the intraoral appliance may be the same as or different from the shape of the inner surface 500 of the intraoral appliance.

In an embodiment, the electronic device 100 may generate an intermediate surface 900 by shifting the inner surface 500 of the intraoral appliance by a predetermined offset value.

For example, the electronic device 100 may shift respective points of the inner surface 500 of the intraoral appliance by a predetermined offset value to identify one or more offset points and, based on the one or more offset points, generate the intermediate surface 900 of the intraoral appliance. That is, the intermediate surface 900 of the intraoral appliance may include one or more offset points.

For example, the electronic device 100 may identify a first point on the inner surface 500 of the intraoral appliance and a normal vector to the inner surface 500 at the first point. The electronic device 100 may identify a first offset point spaced apart from the first point by a first offset value in the direction of the identified normal vector. Here, the first offset value may be a value smaller than the minimum distance between one or more points constituting the inner surface 500 and one or more points constituting the outer surface 600. The electronic device 100 may identify one or more first offset points by applying the first offset value to all points on the inner surface 500 of the intraoral appliance.

For example, based on whether one or more first offset points are located between the inner surface 500 and the outer surface 600 of the intraoral appliance and whether the minimum distance between the one or more first offset points and one or more points constituting the outer surface 600 is greater than a predetermined value, the electronic device 100 may identify second points of the intermediate surface 900 of the intraoral appliance corresponding to the respective points of the inner surface 500 of the intraoral appliance. Here, the predetermined value compared to the minimum distance between the one or more first offset points and one or more points constituting the outer surface 600 may be a reference distance configured to prevent the outer layer of the intraoral appliance formed by the intermediate surface 900 and the outer surface 600 from being formed thinly or to prevent the intermediate surface 900 and the outer surface 600 from partially overlapping. For example, the reference distance may be predetermined. As a specific example, if the minimum distance between one or more points constituting the inner surface 500 and one or more points constituting the outer surface 600 is 0.2 mm, the reference distance may be preset to 0.1 mm, which is half of the minimum distance. As another example, the reference distance may be set by the user 10. As a specific example, the electronic device 100 may receive an input for the reference distance from the user 10.

For example, in response to one or more first offset points being located between the inner surface 500 and the outer surface 600 of the intraoral appliance and to the minimum distance between one or more first offset points and one or more points constituting the outer surface 600 being greater than a predetermined value, the electronic device 100 may identify one or more second points on the intermediate surface 900 of the intraoral appliance as being identical to one or more first offset points. That is, the intermediate surface 900 of the intraoral appliance may be generated such that it is configured as one or more first offset points.

For example, in response to one or more first offset points being located between the inner surface 500 and the outer surface 600 of the intraoral appliance and to the minimum distance between one or more first offset points and one or more points constituting the outer surface 600 being less than or equal to a predetermined value, the electronic device 110 may identify a second offset point that is spaced apart from the first offset point by a second offset value in a direction opposite to the direction of the normal vector to the first offset point at a point on the inner surface 500 of the intraoral appliance corresponding to the first offset point. The electronic device 100 may identify one or more second offset points by applying a second offset value to the first offset point whose minimum distance from one or more points constituting the outer surface 600 is less than or equal to a predetermined value. Here, the second offset value may be a value that makes the minimum distance between the first offset point and one or more points of the outer surface 600 greater than a predetermined value. Alternatively, the electronic device 100 may identify one or more second offset points by applying the second offset value to all of the first offset points. Based on whether one or more second offset points are located between the inner surface 500 and the outer surface 600 of the intraoral appliance and whether the minimum distance between one or more second offset points and one or more points constituting the outer surface 600 is greater than a predetermined value, the electronic device 100 may identify points of the intermediate surface 900 corresponding to the respective points on the inner surface 500 of the intraoral appliance.

For example, in response to at least some of the one or more first offset points not being located between the inner surface 500 and the outer surface 600 of the intraoral appliance, the electronic device 100 may identify a second offset point that is spaced apart from the first offset point by a third offset value in a direction opposite to the direction of the normal vector to the first offset point at a point on the inner surface 500 of the intraoral appliance, which corresponds to the first offset point that is not located between the inner surface 500 and the outer surface 600 of the intraoral appliance. Here, the third offset value may be a value that allows the first offset point to be positioned between the inner surface 500 and the outer surface 600 of the intraoral appliance. Alternatively, the third offset value may be a value that allows the first offset point to be positioned between the inner surface 500 and the outer surface 600 of the intraoral appliance while ensuring that the minimum distance between the first offset point and one or more points on the outer surface 600 is greater than a predetermined value. The electronic device 100 may identify one or more second offset points by applying the third offset value to all the first offset points that are not located between the inner surface 500 and the outer surface 600 of the intraoral appliance. Alternatively, the electronic device 100 may also identify one or more second offset points by applying the third offset value to all of the first offset points. Based on whether all second offset points are located between the inner surface 500 and the outer surface 600 of the intraoral appliance and whether the minimum distance between one or more second offset points and one or more points constituting the outer surface 600 is greater than a predetermined value, the electronic device 100 may identify points on the intermediate surface 900 corresponding to the respective points on the inner surface 500 of the intraoral appliance.

As described above, the electronic device 100 may perform an operation of identifying offset points until all offset points are located between the inner surface 500 and the outer surface 600 of the intraoral appliance and the minimum distance between all offset points and one or more points constituting the outer surface 600 is greater than a predetermined value, thereby identifying all points of the intermediate surface 900 corresponding to the respective points of the inner surface 500 of the intraoral appliance. Therefore, as illustrated in FIG. 9B, a specific point of the inner surface 500 of the intraoral appliance and a specific point of the intermediate surface 900 corresponding thereto may be separated by a predetermined offset value.

For example, the offset value indicating the separation distance between two different points may be configured by the user 10. Here, the offset value may include the first offset value, the second offset value, the third offset value, or the like described in the above example. As a specific example, the electronic device 100 may receive an input for the first offset value from the user 10 through the input device 109. Alternatively, the electronic device 100 may receive an input for the second offset value from the user 10 through the input device 109. Alternatively, the electronic device 100 may receive an input for the third offset value from the user 10 through the input device 109.

As another example, the offset value indicating the separation distance between two different points may be determined by the electronic device 100. As a specific example, the electronic device 100 may identify a plurality of point pairs based on all points on the inner surface 500 of the intraoral appliance and all points on the outer surface 600 of the intraoral appliance. Here, the respective points on the inner surface 500 of the intraoral appliance may have a one-to-one correspondence with the respective points on the outer surface 600. Here, each point pair may include one point on the inner surface 500 of the intraoral appliance and one point on the outer surface 600 corresponding thereto. The electronic device 100 may identify the point pair having the closest distance between the points from among the plurality of point pairs. Thereafter, the electronic device 100 may determine an offset value based on the point pair having the closest distance between the points. Here, the offset value may include the first offset value of the example described above. For example, when a plurality of point pairs are expressed as (the point (Pᵢ) of the inner surface 500 and the point (Pₒ) of the outer surface 600), examples may include (Pᵢ₁, Pₒ₁), (Pᵢ₂, Pₒ₂), and (Pᵢ₃, Pₒ₃). The electronic device 100 may calculate distance 1 (the distance between Pᵢ₁ and Pₒ₁), distance 2 (the distance between Pᵢ₂ and Pₒ₂), and distance 3 (the distance between Pᵢ₃ and Pₒ₃), and determine the shortest distance between the points constituting the point pair as the offset value.

In an embodiment, the electronic device 100 may generate an intermediate surface 900 by identifying intermediate points between the inner surface 500 and the outer surface 600 of the intraoral appliance.

For example, the electronic device 100 may identify a plurality of point pairs, based on all points on the inner surface 500 and all points on the outer surface 600 of the intraoral appliance. Here, the respective points on the inner surface 500 of the intraoral appliance may have a one-to-one correspondence with the respective points on the outer surface 600. Here, each point pair may include one point on the inner surface 500 of the intraoral appliance and one point on the outer surface 600 corresponding thereto. The electronic device 100 may identify a plurality of intermediate points based on the plurality of point pairs. Here, each intermediate point may be located between one point on the inner surface 500 and one point on the outer surface 600 of the intraoral appliance, which constitute each point pair. As a specific example, each intermediate point may be an intermediate value between one point on the inner surface 500 and one point on the outer surface 600 of the intraoral appliance, which constitute each point pair. The electronic device 10 may generate an intermediate surface 900 based on the plurality of intermediate points. That is, the intermediate surface 900 of the intraoral appliance may be configured as a plurality of intermediate points. When a plurality of point pairs are expressed as (the point (Pᵢ) of the inner surface 500 and the point (Pₒ) of the outer surface 600), examples may include (Pᵢ₁, Pₒ₁), (Pᵢ₂, Pₒ₂), and (Pᵢ₃, Pₒ₃). In this case, the electronic device 100 may identify Pₘ₁ which is located between Pᵢ₁ and Pₒ₁, Pₘ₂ which is located between Pᵢ₂ and Pₒ₂, and Pₘ₃ which is located between Pᵢ₃ and Pₒ₃. By identifying Pₘ₁, Pₘ₂, and Pₘ₃, the electronic device 100 may generate an intermediate surface 900 including these points.

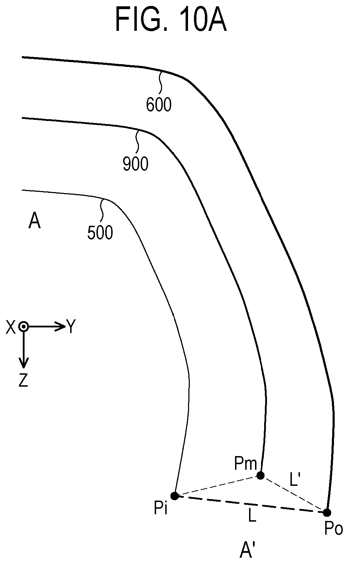

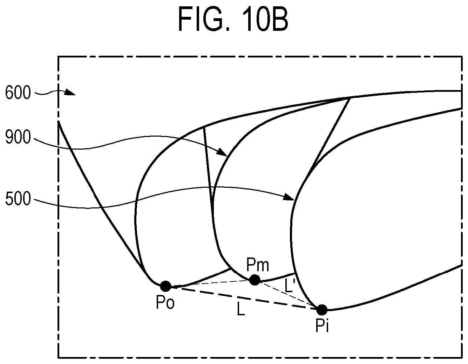

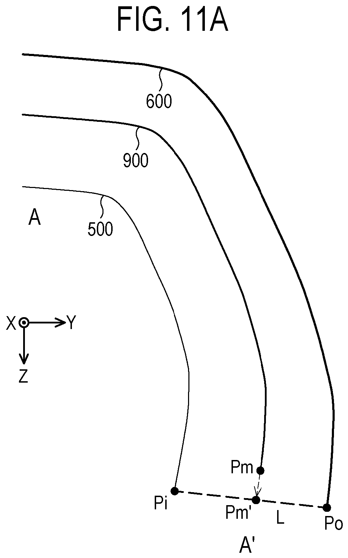

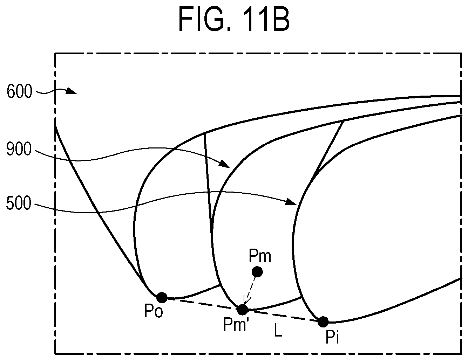

In an embodiment, the electronic device 100 may modify third data on the intermediate surface 900 of the intraoral appliance. Here, the operation of modifying the third data on the intermediate surface 900 may be expressed as an operation of modifying the intermediate surface 900. Hereinafter, the necessity and method of modifying the intermediate surface 900 of the intraoral appliance will be described in detail with reference to FIGS. 10A, 10B, 11A, and 11B.

FIGS. 10A and 10B are diagrams illustrating a periphery of an intermediate surface 900 of the intraoral appliance according to an embodiment of the present disclosure. FIG. 10A is a two-dimensional cross-sectional view of the inner surface 500, the intermediate surface 900, and the outer surface 600 of the intraoral appliance taken along line A-A', and illustrates an example of a bend formed on the periphery of the intermediate surface 900. FIG. 10B is a three-dimensional drawing of the inner surface 500, the intermediate surface 900, and the outer surface 600 of an intraoral appliance, and illustrates an example of a bend formed on the periphery of the intermediate surface 900.

Referring to FIGS. 6, 9A, and 9B, the outer surface 600 and intermediate surface 900 of the intraoral appliance may be generated using different methods, based on the same inner surface 500. For example, referring to FIG. 6, the generation process of the outer surface 600 may include a correction process such as smoothing, while the generation process of the intermediate surface 900 may not include such a correction process. Accordingly, one or more points constituting the periphery of the inner surface 500, one or more points constituting the periphery of the outer surface 600, and one or more points constituting the periphery of the intermediate surface 900 may not be aligned in a straight line. Referring to the examples in FIGS. 10A and 10B, when the electronic device 100 generates the intermediate surface 900 of the intraoral appliance, a specific point Pᵢ of the inner surface 500 of the intraoral appliance and a specific point Pₒ of the outer surface 600 corresponding thereto are located on a single straight line L, but a specific point Pₘ of the intermediate surface 900 is not located on the corresponding straight line L, and may be connected to the specific point Pᵢ of the inner surface 500 of the intraoral appliance and the specific point Pₒ of the outer surface 600 of the intraoral appliance by a bent line L'. In this state, when all points on the inner surface 500 of the intraoral appliance and all corresponding points on the intermediate surface 900 are connected to each other, and all points on the intermediate surface 900 and all corresponding points on the outer surface 600 are connected to each other, a bend will occur in at least a portion of the intraoral appliance, as illustrated in FIGS. 10A and 10B, and such a bend may deteriorate the wearing comfort of the intraoral appliance.

FIGS. 11A and 11B are drawings illustrating a method for modifying the intermediate surface 900 of an intraoral appliance according to an embodiment of the present disclosure. FIG. 11A is a two-dimensional cross-sectional view of the inner surface 500, the intermediate surface 900, and the outer surface 600 of the intraoral appliance taken along line A-A', and illustrates an example in which the intermediate surface 900 of the intraoral appliance is modified to have no bends on the periphery. FIG. 11B is a three-dimensional drawing illustrating the inner surface 500, the intermediate surface 900, and the outer surface 600 of an intraoral appliance, and illustrates an example in which the intermediate surface 900 of the intraoral appliance is modified to have no bends on the periphery of the intermediate surface 900.

In an embodiment, the electronic device 100 may identify a point Pᵢ having the shortest distance from a specific point Pₘ on the periphery of the intermediate surface 900 from among one or more points constituting the periphery of the inner surface 500. Additionally, the electronic device 100 may identify a point Pₒ having the shortest distance from a specific point Pₘ on the periphery of the intermediate surface 900 from among one or more points constituting the periphery of the outer surface 600. The electronic device 100 may modify the intermediate surface 900 so that the intermediate surface 900 includes a specific point P_{m'} located on a straight line L connecting a specific point Pᵢ on the periphery of the inner surface 500 and a specific point Pₒ on the periphery of the outer surface 600. Accordingly, the specific point P_{m'} included in the intermediate surface 900 constitutes the periphery of the intermediate surface 900. The electronic device 100 may perform the above operation on at least some of all points on the periphery of the intermediate surface 900 of the intraoral appliance, thereby positioning the points on the periphery of the intermediate surface 900 of the intraoral appliance on a straight line connecting the points on the inner surface 500 and the points on the periphery of the outer surface 600.

For example, the electronic device 100 may identify a point P_{m'} that is located on a straight line L connecting a specific point Pᵢ on the periphery of the inner surface 500 and a specific point Pₒ on the periphery of the outer surface 600, and spaced apart from the specific point Pᵢ on the periphery of the inner surface 500 by a fourth offset value. The electronic device 100 may modify the intermediate surface 900 so that the intermediate surface 900 includes the identified point P_{m'}. Accordingly, the specific point P_{m'} included in the intermediate surface 900 constitutes the periphery of the intermediate surface 900. The electronic device 100 performs the above operation on at least some of all points on the periphery of the intermediate surface 900 of the intraoral appliance, thereby positioning the points on the periphery of the intermediate surface 900 on a straight line connecting the points on the periphery of the inner surface 500 and the points on the periphery of the outer surface 600. Meanwhile, the fourth offset value may be configured by the user 10. As a specific example, the electronic device 100 may receive a fourth offset value from the user 10 via the input device 109.

In an embodiment, the electronic device 100 may receive, from the user 10, an input to move a specific point Pₘ on the periphery of the intermediate surface 900 of the intraoral appliance via the input device 109. Based on the input from the user 10, the electronic device 100 may identify a point P_{m'} that is located on a straight line connecting a specific point Pᵢ of the inner surface 500 of the intraoral appliance and a specific point Pₒ of the outer surface 600, and spaced apart from the specific point Pᵢ of the inner surface 500. The electronic device 100 may modify the intermediate surface 900 so that the intermediate surface 900 includes the identified point P_{m'}. Accordingly, the specific point P_{m'} included in the intermediate surface 900 constitutes the periphery of the intermediate surface 900. Here, the input of the user 10 for moving the specific point Pₘ of the intermediate surface 900 of the intraoral appliance may include three-dimensional coordinates of a point P_{m'} that is located on a straight line connecting a specific point Pᵢ of the inner surface 500 and a specific point Pₒ of the outer surface 600, and spaced apart from the specific point Pᵢ of the inner surface 500.

By modifying the intermediate surface 900 of the intraoral appliance so that the points on the periphery of the intermediate surface 900 are positioned on a straight line connecting the points on the periphery of the inner surface 500 and the points on the periphery of the outer surface 600 of the intraoral appliance, the inner surface 500, the outer surface 600, and the intermediate surface 900 of the intraoral appliance may be smoothly connected without any bends, forming a sufficiently wide and flat contact region between the intraoral appliance and the gingival region, which improves the wearing comfort of the intraoral appliance and enhances the aesthetic value perceived by the wearer of the intraoral appliance. Further, as described below, even when two or more materials are applied to the intraoral appliance, the wearer of the intraoral appliance may substantially avoid feeling a sense of foreignness in the region where the intraoral appliance and a portion of the intraoral structure come into contact.

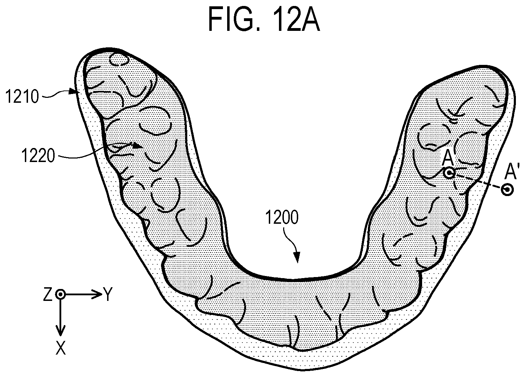

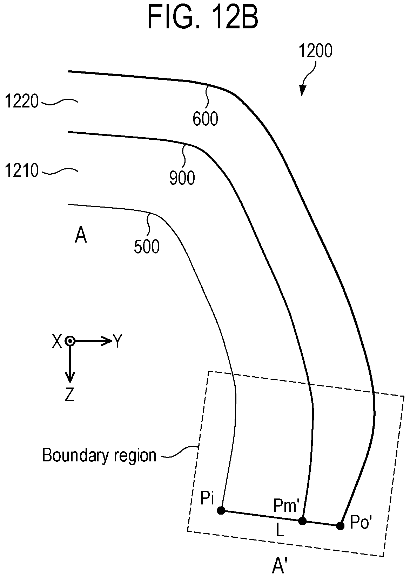

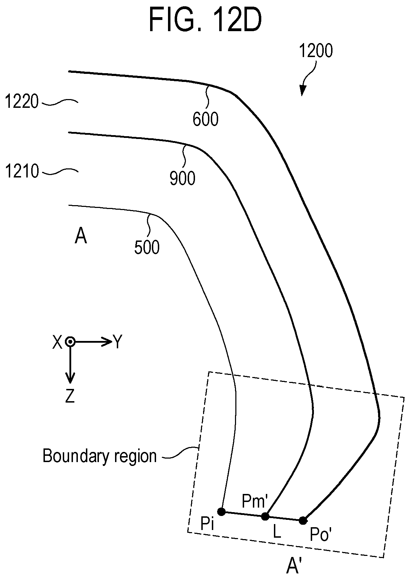

FIGS. 12A and 12D are diagrams illustrating an intraoral appliance 1200 according to an embodiment of the present disclosure. FIG. 12A is a three-dimensional drawing of an intraoral appliance 1200, and FIGS. 12B to 12D are cross-sectional views of the intraoral appliance 1200 taken along line A-A'.

Referring to FIG. 12A, the intraoral appliance 1200 may include a first portion 1210 and a second portion 1220.

In an embodiment, the electronic device 100 may identify a region between the inner surface 500 and the intermediate surface 900 of the intraoral appliance 1200 as a first portion 1210 of the intraoral appliance 1200.

For example, the first portion 1210 of the intraoral appliance 1200 may be an inner layer of the intraoral appliance 1200 and may accommodate at least one tooth within a teeth region located in a direction in which the intraoral appliance 1200 is inserted into the oral cavity.

For example, the first portion 1210 of the intraoral appliance 1200 may be a region formed by connecting all points on the inner surface 500 of the intraoral appliance 1200 and all points on the intermediate surface 900.

For example, the electronic device 100 may identify a first material as the material of the first portion 1210 of the intraoral appliance 1200. Here, the first material may be a transparent material. Further, the first material may be a material having flexible physical properties (flexibility). For example, the first material may be a material having a high elongation (e.g., 45 to 55%). Meanwhile, the physical properties of the first material may be determined by various criteria, such as elongation, and the embodiments of the present disclosure may be applied in the same manner.

In an embodiment, the electronic device 100 may identify a region between the intermediate surface 900 and the outer surface 600 of the intraoral appliance 1200 as a second portion 1220 of the intraoral appliance 1200.

For example, the second portion 1220 of the intraoral appliance 1200 may be an outer layer of the intraoral appliance 1200 and may form an outline of the intraoral appliance 1200.

For example, the second portion 1220 of the intraoral appliance 1200 may be a region formed by connecting all points of the intermediate surface 900 and all points of the outer surface 600 of the intraoral appliance 1200.

For example, the electronic device 100 may identify a second material as the material of the second portion 1220 of the intraoral appliance 1200. Here, the second material may be a transparent material. Further, the second material may be a material with hard physical properties. For example, the second material may be a material with low elongation (e.g., less than 45 to 55%). Meanwhile, the physical properties of the second material may be determined by various criteria, such as elongation, and the embodiments of the present disclosure may be applied in the same manner.

For example, the first material and the second material may have different physical properties. As a specific example, the first material may be more flexible than the second material. That is, the first material may be a material with more flexible physical properties, such as a high elongation, than the second material. Alternatively, the first material may be a smooth material with less roughness than the second material. In this way, the first portion 1210 of the intraoral appliance 1200 is made of a softer and more flexible material than the second portion 1220, thereby further improving the wearing comfort of the intraoral appliance 1200. Furthermore, the second portion 1220 of the intraoral appliance 1200 is made of a harder material than the first portion 1210, thereby ensuring that the intraoral appliance 1200 maintains its shape for a long time with high durability even when the wearer of the intraoral appliance 1200 experiences strong bruxism. As described above, by applying different materials to a portion of the intraoral appliance 1200, both the wearing comfort and durability may be improved, compared to applying a single material to the intraoral appliance 1200.

As illustrated in FIGS. 12B to 12D, a boundary region (dotted lines) may be configured for the inner surface 500, the outer surface 600, and the intermediate surface 900 of the intraoral appliance 1200. For example, the boundary region may be predetermined by factors such as a buccal height, a lingual height, etc. Alternatively, the boundary regions may be configured by the user 10. For example, the electronic device 100 may receive input for the boundary region from the user 10 via the input device 109.

In an embodiment, a thickness of at least one of the first portion 1210 or the second portion 1220 of the intraoral appliance 1200 may be reduced as it approaches the gingival region in the boundary region.

Referring to FIG. 12B, the distance between the vertex of the intermediate surface 900 and the vertex of the outer surface 600 within the boundary region may become shorter as it approaches the outline. That is, the second portion 1220 of the intraoral appliance 1200 may become thinner as it approaches the gingival region in the oral cavity.

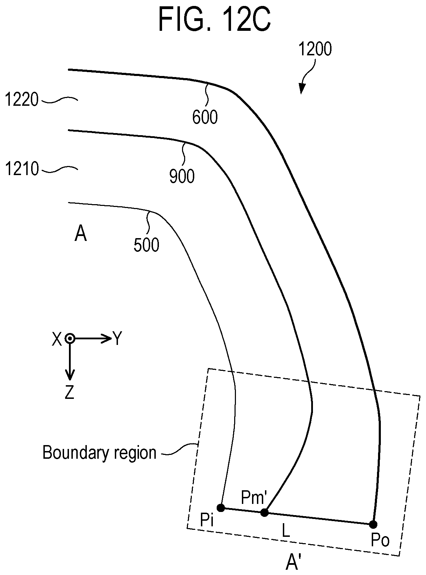

Referring to FIG. 12C, the distance between the vertex of the inner surface 500 and the vertex of the intermediate surface 900 within the boundary region may become shorter as it approaches the outline. That is, the first portion 1210 of the intraoral appliance 1200 may become thinner as it approaches the gingival region in the oral cavity.

Referring to FIG. 12D, the distance between the vertex of the inner surface 500 and the vertex of the intermediate surface 900 within the boundary region, and the distance between the vertex of the intermediate surface 900 and the vertex of the outer surface 600 may become shorter as it approaches the outline. That is, both the first portion 1210 and the second portion 1220 of the intraoral appliance 1200 may become thinner toward the gingival region.

In an embodiment, the electronic device 100 may determine whether to thin the first portion 1210 or the second portion 1220 of the intraoral appliance 1200 as it approaches the gingival region within the boundary region. For example, the electronic device 100, based on the area of the contact region between the intraoral appliance 1200 and the intraoral gingival region, may determine whether to thin the first portion 1210 or the second portion 1220 as it approaches the gingival region within the boundary region. When the electronic device 100 determines to thin the second portion 1220 as it approaches the gingival region within the boundary region, reference may be made to FIG. 12B. When the electronic device 100 determines to thin the first portion 1210 as it approaches the gingival region within the boundary region, reference may be made to FIG. 12C. When the electronic device 100 determines to thin both the first portion 1210 and the second portion 1220 as they approach the gingival region within the boundary region, reference may be made to FIG. 12D.

In an embodiment, the user 10 may determine which of the first portion 1210 and the second portion 1220 of the intraoral appliance 1200 is to be thinner as they approach the gingival region within the boundary region. The electronic device 100 may receive an input for selecting at least one of the first portion 1210 or the second portion 1220 of the intraoral appliance 1200 from the user 10 via the input device 109. When the user 10 selects the second portion 1220, reference may be made to FIG. 12B. When the user 10 selects the first portion 1210, reference may be made to FIG. 12C. When the user 10 selects both the first portion 1210 and the second portion 1220, reference may be made to FIG. 12D.

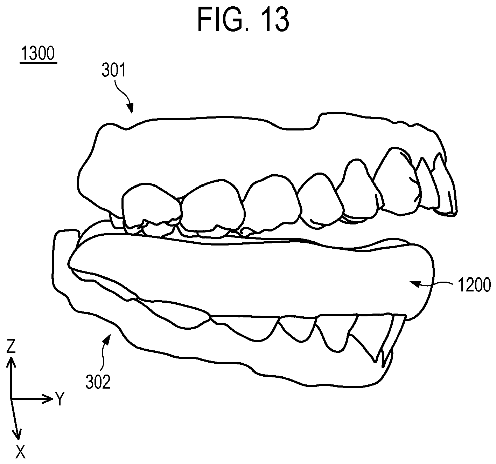

FIG. 13 is a drawing illustrating a state 1300 in which an intraoral appliance 1200 is mounted on a subject 20 according to an embodiment of the present disclosure.

Referring to FIG. 13, the intraoral appliance 1200 may be mounted on the mandible 302 of the intraoral structure of the subject 20. Meanwhile, this is merely an example for explaining an embodiment of the present disclosure, and the present disclosure is not limited thereto. According to an embodiment of the present disclosure, an intraoral appliance 1200 capable of being mounted on the maxilla 301 may be generated, and an intraoral appliance 1200 capable of being mounted simultaneously on both of the bimaxillary portions 301 and 302 may also be generated.

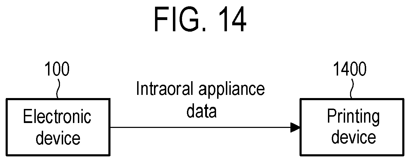

Meanwhile, the intraoral appliance 1200 may also be generated by an external device, such as a printing device. This will be described in detail with reference to FIG. 14.

FIG. 14 is a diagram illustrating a method for generating (printing) an intraoral appliance 1200 according to an embodiment of the present disclosure.

In an embodiment, the electronic device 100 may transmit data on the intraoral appliance 1200 to a printing device 1400.

For example, the printing device 1400 may include a three-dimensional printing device capable of generating (printing) a physical form of an object represented by the received data. Here, the three-dimensional printing device may include a three-dimensional printing device capable of generating an object using a single material, a three-dimensional printing device capable of generating an object using two or more materials (multijet method), and the like. The printing device 1400 may generate (print) an intraoral appliance 1200 based on the received data.

For example, data on the intraoral appliance 1200 may include at least one piece of the following data or a combination thereof.
- First data on the inner surface 500 of the intraoral appliance 1200
- Second data on the outer surface 600 of the intraoral appliance 1200
- Third data on the intermediate surface 900 of the intraoral appliance 1200
- Fourth data on the first portion 1210 of the intraoral appliance 1200
- Fifth data on the second portion 1220 of the intraoral appliance 1200
- Sixth data on the overall shape of the intraoral appliance 1200

For example, when the data on the intraoral appliance 1200 includes the first data on the inner surface 500, the second data on the outer surface 600, and the third data on the intermediate surface 900, the printing device 1400, based on the received first data, second data, and third data, may identify the first portion 1210 and the second portion 1220 of the intraoral appliance 1200. The printing device 1400 may identify the first material as the first substance of the first portion 1210 of the intraoral appliance 1200 and the second material as the second substance of the second portion 1220. Thereafter, the printing device 1400 may apply the first substance configured as the first material to the first portion 1210 of the intraoral appliance 1200 and apply the second substance configured as the second material to the second portion 1220, thereby generating (printing) the intraoral appliance 1200. Alternatively, the printing device 1400 may generate (print) the first portion 1210 of the intraoral appliance 1200 by applying the first substance configured as the first material, and generate (print) the second portion 1220 by applying the second substance configured as the second material. That is, the printing device 1400 may separately generate (print) the first portion 1210 and the second portion 1220 of the intraoral appliance 1200. In this case, the first portion 1210 of the intraoral appliance 1200 may be utilized as a dental aligner. As a specific example, if the first portion 1210 of the intraoral appliance 1200 is generated by applying a transparent material, the first portion 1210 of the intraoral appliance 1200 may be utilized as a dental aligner. Alternatively, it is also possible to lock the first portion 1210 and second portion 1220 of the intraoral appliance 1200, which are separately generated, and utilize them as a splint. Meanwhile, when locking the first portion 1210 and second portion 1220 of the intraoral appliance 1200, the locking angle and clearance between the first portion 1210 and second portion 1220 must be considered. If the materials applied to the first portion 1210 and the second portion 1220 differ from each other, then even if the first portion 1210 and second portion 1220 are precisely matched in the data, a crack (gap) may be formed between the first portion 1210 and second portion 1220 in actual fabrication due to material characteristics (e.g., particle size, etc.), which may hinder proper locking. In this case, correction values, such as the locking angle and clearance for the locking of the first portion 1210 and the second portion 1220 need to be further considered. Meanwhile, since the first portion 1210 of the intraoral appliance 1200 is formed by the inner surface 500 and the intermediate surface 900 of the intraoral appliance 1200, and the second portion 1220 of the intraoral appliance 1200 is formed by the intermediate surface 900 and the outer surface 600 of the intraoral appliance, data including correction values, such as the locking angle of the first portion 1210 and the second portion 1220 of the intraoral appliance 1200, may be identified in the process of generating the intermediate surface 900, which serves as the boundary between the first portion 1210 and the second portion 1220 of the intraoral appliance. Accordingly, the third data on the intermediate surface 900 of the intraoral appliance 1200 may include data for locking the first portion 1210 and the second portion 1220 of the intraoral appliance 1200. The printing device 1400 may further correct and separately generate (print) the first portion 1210 and the second portion 1220 of the intraoral appliance 1200, based on the data for locking the first portion 1210 and the second portion 1220 of the intraoral appliance 1200. Alternatively, the printing device 1400 may correct the already generated first portion 1210 and second portion 1220 of the intraoral appliance 1200, based on the data for locking the first portion 1210 and the second portion 1220 of the intraoral appliance 1200. In this way, the operation of correcting the already generated first portion 1210 and second portion 1220 of the intraoral appliance 1200 may be expressed as a post-processing operation.

For example, in the case where the data on the intraoral appliance 1200 includes fourth data on the first portion 1210, the printing device 1400, based on the received fourth data, may identify the first portion 1210 of the intraoral appliance 1200 and identify the first material as the first substance of the first portion 1210. Here, the fourth data on the first portion 1210 may also include data on the first substance of the first portion 1210. Thereafter, the printing device 1400 may generate (print) the first portion 1210 of the intraoral appliance 1200 by applying the first substance configured as the first material.

In the case where the data on the intraoral appliance 1200 includes fifth data on the second portion 1220 by way of example, the printing device 1400, based on the received fifth data, may identify the second portion 1220 of the intraoral appliance 1200 and identify the second material as the second substance of the second portion 1220. Here, the fifth data on the second portion 1220 may also include data on the second substance of the second portion 1220. Thereafter, the printing device 1400 may generate (print) the second portion 1220 of the intraoral appliance 1200 by applying the second substance configured as the second material.

In the case where data on an intraoral appliance 1200 includes fourth data on the first portion 1210 and fifth data on the second portion 1220 by way of example, the printing device 1400, based on the received fourth data and fifth data, may identify the first portion 1210 and the second portion 1220 of the intraoral appliance 1200. The printing device 1400 may identify the first material as the first substance of the first portion 1210 and the second material as the second substance of the second portion 1220. Thereafter, the printing device 1400 may apply the first substance configured as the first material to the first portion 1210 and the second substance configured as the second material to the second portion 1220, thereby generating (printing) the intraoral appliance 1200. Alternatively, the printing device 1400 may generate (print) the first portion 1210 of the intraoral appliance 1200 by applying the first substance configured as the first material, and generate (print) second portion 1220 by applying the second substance configured as the second material. That is, the printing device 1400 may separately generate (print) the first portion 1210 and the second portion 1220 of the intraoral appliance 1200. In this case, the electronic device 100 may transmit data for locking the first portion 1210 and the second portion 1220 of the intraoral appliance 1200 to the printing device 1400. For example, the data for locking the first portion 1210 and the second portion 1220 of the intraoral appliance 1200 may be included in at least some of the fourth data on the first portion 1210 and the fifth data on the second portion 1220. The printing device 1400 may further correct and separately generate (print) the first portion 1210 and the second portion 1220 of the intraoral appliance 1200 according to the data for locking the first portion 1210 and the second portion 1220 of the intraoral appliance 1200. Alternatively, the printing device 1400 may correct the already generated first portion 1210 and second portion 1220 of the intraoral appliance 1200, based on the data for locking the first portion 1210 and the second portion 1220 of the intraoral appliance 1200.

As described above, the electronic device 100 may transmit, to the printing device 1400, the first data on the inner surface 500, the second data on the outer surface 600, the third data on the intermediate surface 900, the fourth data on the first portion 1210, and the fifth data on the second portion 1220, as intermediate data for generating (printing) the intraoral appliance 1200, but the electronic device 100 may transmit, to the printing device 1400, the sixth data on the overall shape of the intraoral appliance 1200 as final data for generating (printing) the intraoral appliance 1200. Here, the sixth data on the overall shape of the intraoral appliance 1200 may be data generated based on the first data, the second data, the third data, the fourth data, or the fifth data.

Although the process steps, method steps, algorithms, and the like have been described in a sequential order in the flowcharts illustrated in this document, such processes, methods, and algorithms may be configured to operate in any suitable order. In other words, the steps of the processes, methods, and algorithms described in various embodiments of the present disclosure need not be performed in the order described in the present disclosure. Further, even if some steps are described as being performed asynchronously, in other embodiments, such steps may be performed simultaneously. Furthermore, the illustration of a process in the drawings does not imply that the illustrated process excludes other variations or modifications. In addition, it also does not imply that the illustrated process or any of its steps is essential to one or more embodiments of the present disclosure, nor that the illustrated process is preferred.

Although the method has been described through specific embodiments, the method may also be implemented as computer-readable code on a computer-readable recording medium. The computer-readable recording medium may include all types of recording devices that store data that can be read by a computer system. Examples of computer-readable recording media may include a ROM, a RAM, a CD-ROM, a magnetic tape, a floppy disk, and an optical data storage devices. Furthermore, computer-readable recording media may be distributed across network-connected computer systems, allowing computer-readable code to be stored and executed in a distributed manner. Functional programs, codes, and code segments for implementing the above embodiments may be readily inferred by programmers skilled in the art to which the present disclosure pertains.

## Claims

1. A method performed by an electronic device, the method comprising:
generating first data on an inner surface of an intraoral appliance, based on three-dimensional scan data on a surface of a subject;
generating second data on an outer surface of the intraoral appliance, based on the first data; and generating third data on an intermediate surface of the intraoral appliance located between the inner surface and the outer surface, based on the first data and the second data.

2. The method of Claim 1, further comprising:
identifying a first material as a substance of a first portion of the intraoral appliance between the inner surface and the intermediate surface; and
identifying a second material as a substance of a second portion of the intraoral appliance between the intermediate surface and the outer surface.

3. The method of Claim 2, wherein the first portion of the intraoral appliance accommodates one or more teeth within an oral cavity,
wherein the second portion of the intraoral appliance forms an outline of the intraoral appliance, and
wherein at least one of the first portion or the second portion of the intraoral appliance becomes thinner toward a gingival region within the oral cavity.

4. The method of Claim 2, wherein the third data further includes data on locking of the first portion and the second portion.

5. The method of Claim 2, wherein the first material is more flexible than the second material.

6. The method of Claim 1, wherein the generating the third data on the intermediate surface comprises:
identifying a first point on the inner surface and a normal vector to the inner surface at the first point;
identifying a first offset point spaced apart from the first point by a first offset value in a direction of the normal vector; and
identifying a second point on the intermediate surface corresponding to the first point, based on whether the first offset point is located between the inner surface and the outer surface and a minimum distance between the first offset point and one or more points on the outer surface is greater than a predetermined value.

7. The method of Claim 6, wherein the identifying the second point on the intermediate surface corresponding to the first point, based on whether the first offset point is located between the inner surface and the outer surface and the minimum distance between the first offset point and the one or more points on the outer surface is greater than the predetermined value, further comprises:
identifying the second point as being identical to the first offset point in response to the first offset point being located between the inner surface and the outer surface and the minimum distance between the first offset point and the one or more points on the outer surface being greater than the predetermined value.

8. The method of Claim 6, wherein the identifying the second point on the intermediate surface corresponding to the first point, based on whether the first offset point is located between the inner surface and the outer surface and the minimum distance between the first offset point and the one or more points on the outer surface is greater than the predetermined value, comprises:
identifying a second offset point spaced apart from the first offset point by a second offset value in a direction opposite to the normal vector in response to the first offset point being located between the inner surface and the outer surface and to the minimum distance between the first offset point and the one or more points of the outer surface being less than or equal to the predetermined value; and
identifying a second point on the intermediate surface corresponding to the first point, based on whether the second offset point is located between the inner surface and the outer surface and a minimum distance between the second offset point and the one or more points on the outer surface is greater than the predetermined value.

9. The method of Claim 6, wherein the identifying the second point on the intermediate surface corresponding to the first point, based on whether the first offset point is located between the inner surface and the outer surface and the minimum distance between the first offset point and the one or more points on the outer surface is greater than the predetermined value, comprises:
identifying a third offset point spaced apart from the first offset point by a third offset value in a direction opposite to the normal vector in response to the first offset point not being located between the inner surface and the outer surface; and
identifying the second point on the intermediate surface corresponding to the first point, based on whether the third offset point is located between the inner surface and the outer surface and a minimum distance between the third offset point and the one or more points on the outer surface is greater than the predetermined value.

10. The method of Claim 6, further comprising:
identifying a plurality of point pairs, each point pair comprising one point on the inner surface and one point on the outer surface;
identifying a point pair having a closest distance between points from among the plurality of point pairs; and
determining the first offset value, based on the identified point pair.

11. The method of Claim 6, further comprising receiving a user input for the first offset value.

12. The method of Claim 6, wherein the generating the third data on the intermediate surface comprises:
identifying a plurality of point pairs, each point pair comprising one point on the inner surface and one point on the outer surface;
identifying a plurality of intermediate points based on the plurality of point pairs, each intermediate point positioned between one point on the inner surface and one point on the outer surface, which constitute a point pair; and
generating the third data such that the intermediate surface includes the plurality of intermediate points.

13. The method of Claim 1, further comprising:
identifying a specific point having a shortest distance from a specific point on a periphery of the intermediate surface from among one or more points constituting a periphery of the inner surface; identifying a specific point having a shortest distance from a specific point on the periphery of the intermediate surface from among one or more points constituting a periphery of the outer surface; and
modifying the third data so that the intermediate surface includes a specific point on a straight line connecting the specific point on the periphery of the inner surface and the specific point on the periphery of the outer surface.

14. The method of Claim 13, wherein the modifying the third data comprises:
identifying a point located on a straight line connecting the specific point on the periphery of the inner surface and the specific point on the periphery of the outer surface, and spaced apart from the specific point on the periphery of the inner surface by a fourth offset value; and
modifying the third data so that the intermediate surface includes the identified point.

15. The method of Claim 1, further comprising:
receiving a user input for moving a specific point on the intermediate surface;
identifying, based on the user input, a point located on a straight line connecting a specific point on a periphery of the inner surface and a specific point on a periphery of the outer surface, and spaced apart from the specific point on the periphery of the inner surface; and
modifying the third data so that the intermediate surface includes the identified point.

16. An electronic device comprising:
one or more processors; and
one or more memories configured to store instructions executed by the one or more processors, wherein, when the instructions are executed by the one or more processors, the one or more processors are configured to execute a method according to any one of Claims 1 to 15.

17. A non-transitory computer-readable recording medium having instructions recorded thereon, which, when executed by one or more processors, cause the one or more processors to perform an operation,
wherein the instructions are configured to cause the one or more processors to execute a method according to any one of Claims 1 to 15.
